# EUROPÄISCHE PATENTANMELDUNG

(11) **EP 2 138 155 A2**
(43) Veröffentlichungstag der Anmeldung: **30.12.2009**
(21) Anmeldenummer: 09161756.3
(22) Anmeldetag: 03.06.2009
(51) Int. Cl.: A61K 8/39, A61K 8/44, A61K 8/63, A61Q 5/12

(54) **Silkonfreie Konditioniermittel für keratinhaltige Fasern**

(30) Priorität: 27.06.2008 DE 102008030131
(71) Anmelder: Beiersdorf AG, 20245 Hamburg (DE)
(72) Erfinder: Demitz, Michael, 22529, Hamburg (DE); Köhler, Manuela, 20144, Hamburg (DE); Mahadeshwar, Anand, 22529, Hamburg (DE); Saladin, Sandra, 20144, Hamburg (DE); Wendicke, Silke, 22303, Hamburg (DE)

(57) **Zusammenfassung**

Silikonfreie kosmetische Haarbehandlungszubereitung vorgesehen für das Ausspülen nach der Anwendung enthaltend PPG-3 Benzyl Ether Myristate dadurch gekennzeichnet, dass 0,0001 bis 1 Gew.-% amphotere Tenside enthalten sind.

## Beschreibung

Haarspülungen werden zur Pflege nach der Reinigung der Haare angewendet. Ein wichtiger Faktor ist die Sensorik der Spülung während des Auftragens auf das Haar. Der Konsument verbindet mit einer reichhaltigen Haarspülung eine sehr gute Pflegewirkung. Viele Haarspülungen zeigen zwar eine gute Leistung hinsichtlich der Pflegeeigenschaften, haben jedoch eine schlechte Sensorik, vor allem im nassen Haar.

"Sensorik" im Sinne dieser Schrift umfasst alle fühlbaren und für den Anwender relevanten Eigenschaften des Produktes bei Entnahme des Produktes aus der Packung, bestimmungsgemäßer Anwendung (Einmassieren in das Haar, Aufenthalt des Produktes im Haar, Ausspülen) sowie die während und nach der Anwendung fühlbaren Veränderungen an Haar, Kopfhaut und Fingern. Insbesondere umfasst "Sensorik" die Eigenschaften "Gehaltvoll", "Gleitvermögen", "Entwirrbarkeit", "Kämmbarkeit", "Haarstruktur", "Weich geschmeidig".

"Gehaltvoll" im Sinne dieser Schrift wird wie folgt festgelegt: Es wird gemessen in welchem Ausmaß das Produkt als Schicht/Film zwischen der Haarsträhne und den Fingern wahrnehmbar ist. Hierbei wird eine 10er Skala verwendet bei der 10 den höchstmöglichen Gehalt darstellt.

"Gleitvermögen" im Sinne dieser Schrift bedeutet das Gleiten der Finger mit mittlerem/gleich bleibenden Druck an der Haarsträhne von oben nach unten. Hierbei wird eine 10er Skala verwendet bei der 10 das höchstmögliche Gleitvermögen darstellt.

"Entwirrbarkeit" im Sinne dieser Schrift ist die Messung wie leicht sich die Haare nach dem Ausspülen des Produkts mit dem Kamm entwirren lassen. Die Haare sind dann entwirrt, wenn der Kamm einmal komplett von oben nach unten durch die Haarsträhne geführt werden kann. Hierbei wird eine 10er Skala verwendet bei der 10 die höchstmögliche Entwirrbarkeit darstellt.

"Kämmbarkeit" im Sinne dieser Schrift ist die Messung wie leicht sich das Haar mit dem Kamm durchkämmen lässt, nachdem die Haare entwirrt wurden. Hierbei wird eine 10er Skala verwendet bei der 10 die höchstmögliche Kämmbarkeit darstellt.

"Haarstruktur" im Sinne dieser Schrift bedeutet die Beurteilung des Haares durch horizontales Rollen der Haare zwischen Daumen und Zeigefinger/ Mittelfinger einer Hand. Dies wird an verschiedenen Stellen der Strähne beginnend von oben nach unten geprüft. Hierbei wird eine 10er Skala verwendet bei der 10 die höchstmögliche Haarstruktur darstellt.

"Weich geschmeidig" im Sinne dieser Schrift bedeutet das sensorische Empfinden nach Applikation des Produktes im nassen und trockenen Haar durch einen Experten Panel.

Kationische Polymere sind Makromolekulare Substanzen, welche in der Haupt- und/oder Seitenkette eine Gruppe aufweisen, welche "temporär" oder "permanent" kationisch sein kann.

Geeignete kationische Polymere sind beispielsweise quaternisierte Cellulose-Derivate, wie sie unter den Bezeichnungen Celquat und Polymer JR im Handel erhältlich sind. Die Verbindungen CelquatH 100, CelquatL 200 und Polymer JR400 sind bevorzugte quaternierte Cellulose-Derivate, kationische Alkylpolyglycoside gemäss der DE 44 13 686, kationisierter Honig, beispielsweise das Handelsprodukt Honeyquat 50, kationische Guar-Derivate, wie insbesondere die unter den Handelsnamen Cosmedia Guar und Jaguarvertriebenen Produkte, polymere Dimethyldiallylammoniumsalze und deren Copolymere mit Estern und Amiden von Acrylsäure und Methacrylsäure. Die unter den Bezeichnungen Merquat 100 (Po-ly(dimethyldiallylammoniumchlorid)) und Merquat550 (Dimethyldiallylammoniumchlorid-Acrylamid-Copolymer) im Handel erhältlichen Produkte sind Beispiele für solche kationischen Polymere, Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat 734 und Gafquat 755 im Handel erhältlich, Vinylpyrrolidon-Vinylimidazoliummethochlorid-Copolymere, wie sie unter den Bezeichnungen Luviquat FC 370, FC 550, FC 905 und HM 552 angeboten werden, quaternierter Polyvinylalkohol, sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette, die unter den Bezeichnungen Polyquaternium-24 (Handelsprodukt z. B. Quatrisoft LM 200), bekannten Polymere, die Copolymere des Vinylpyrrolidons, wie sie als Handelsprodukte Copolymer 845 (Hersteller: ISP), Gaffix VC 713 (Hersteller: ISP), Gafquat ASCP 1011, GafquatHS 110, Luviquat8155 und Luviquat MS 370 erhältlich sind.

EP1435978A2 offenbart die Verwendung von Estern von alkoxylierten Alkoholen und Fettcarbonsäuren kosmetischen Zubereitungen.

Eine Haarspülung soll aus Konsumentensicht reichhaltig und cremig sein, das Haar pflegen, das Haar im nassen und trockenen Zustand weich, geschmeidig, gut kämmbar, glänzend machen und die Spülung muss schnell wirken.

Die Nutzung von Silikonen in Personal Care Produkten ist weit verbreitet. In Haarspülungen sorgen Silikone für verschiedene verbraucherrelevante Benefits wie z. B. Geschmeidigkeit, gute Kämmbarkeit und Glanz. Jedoch kann es bei der Verwendung von Silikonen auch Nachteile geben, unter anderem kann es zu einem schnelleren Nachfetten der Haare kommen oder das Volumen vermindern. Dies geschieht durch die Filmbildungseigenschaften der Silikone insbesondere bei höheren Einsatzkonzentrationen (z.B. bei Produkten für trocken/strapaziertes Haar).

Es hat sich überraschend gezeigt, dass silikonfreie kosmetische Haarbehandlungszubereitung vorgesehen für das Ausspülen nach der Anwendung enthaltend PPG-3 Benzyl Ether Myristate ben Nachteilen des Standes der Technik abhelfen.

Silikonfrei bedeutet einen Gehalt an höchstens 0,001 Gew.-% Silikonöl. Dabei ist es von Vorteil, wenn die Zubereitung einen Gehalt an PPG-3 Benzyl Ether Myristate von 0,5 bis 4 Gew.-%, bevorzugt 1 bis 3 Gew.-% aufweist.

Durch die Verwendung eines Esters von einem alkoxylierten aromatischen Alkohol und Fettcarbonsäure (PPG-3 Benzyl Ether Myristate, Crodamol STS) als Emollient anstelle eines Silikons oder mehrerer Silikone, ist es möglich die gleiche Pflege-Performance wie mit Silikone zu erzielen, jedoch mit einer geringeren Einsatzkonzentration. Weiterhin zeigten Formeln mit Crodamol STS Vorteile gegenüber Silikone bez. Volumen und schnellere Nachfettung. Diese wurde in einem Verbraucher In-Use-Test mit 60 Personen mit trocken/strapazierten Haaren gezeigt.

Dabei ist es weiter von Vorteil, wenn zusätzlich 0,1 bis 1 Gew.-% Oryzanol enthalten sind. Dabei ist es weiter von Vorteil, wenn 0,0001 bis 1 Gew.-% amphotere Tenside und/oder Amidoamine enthalten sind. Dabei ist es weiter von Vorteil, wenn die Zubereitung frei von Cellulosederivaten ist. Dabei ist es weiter von Vorteil, wenn die Zubereitung nicht waschaktiv ist. Dabei ist es weiter von Vorteil, wenn Zubereitung als amphotere Tenside Betaine, ganz besonders bevorzugt Cocobetaine enthält.

Erfindungsgemäße Zubereitungen können zusätzlich UV- Filter, Polyole, Konservierungsmittel, hydrolysierte Proteine, Pflanzenextrakte, Parfümöle, und Antioxidantien sowie auch amphotere Tenside enthalten.

Besonders bevorzugte erfindungsgemäße Mittel Fasern sind **dadurch gekennzeichnet, dass** sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel Coenzym Q10 enthalten.

Alternativ zu den besonders bevorzugten Ubichinonen oder zusätzlich zu ihnen können die erfindungsgemäßen Mittel auch Plastochinone enthalten. Hier sind bevorzugte erfindungsgemäße Mittel **dadurch gekennzeichnet, dass** sie 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinons, besonders bevorzugt Mittel Plastochinon PQ-9 enthalten.

Es ist bekannt dass besonders tertiäre Amine in Kombination mit den Fettalkoholen Cetyl und Stearyl Alkoholen zu reichhaltigen, cremigen Spülungsgrundlagen führen, wie in EP 0859587B1, EP617953B1, W02002102334A2 und EP1586298B1 beschrieben.

Spülungsformeln 1-3 (Tabelle 1) wurden in einem Verbraucheraktzeptanztest mit 60 Probanden mit trocken/strapazierten Haaren evaluiert. Die Spülungen wurden 2 Wochen lang verwendet. Aus Tabelle 2 erkennt man, dass die Spülungsformeln 2 & 3 besser evaluiert worden sind (höhere Werte) als Formel 1 bezüglich folgender Parameter: Volumen, nicht fettiges Aussehen, keine Beschwerung und nass Kämmbarkeit. Dabei wurde Formel 3 signifikant höher bewertet als 1 & 2. Die Formeln 2 & 3 enthalten 2% aktiv Crodamol STS und enthalten keine Silikone, während Formel 1 eine Gesamtkonzentration an Silikone von 3% enthält, bestehend aus 2% Cyclomethicone und 1% Dimethicone (DC 200 Fluid 10cst).

| | wt % | **1** | **2** | **3** |
|---|---|---|---|---|
| 1 | Methylparaben | 0,25 | 0,25 | 0,25 |
| 2 | Propylparaben | 0,10 | 0,10 | 0,10 |
| 3 | Aqua | 78,36 | 87,83 | 81,74 |
| 4 | Citric Acid | 0,01 | | |
| 5 | Cetearyl Alcohol | | | 5,24 |
| 6 | Cetyl Alcohol | 2,00 | 2,00 | |
| 7 | **Dimethicone** | **1,00** | | |
| 8 | Lactic Acid | | 0,80 | |
| 9 | Benzophenone-4 | 0,25 | | 0,25 |
| 10 | Glycerin | 5,00 | | 5,00 |
| 11 | Sodium Hydroxide | 0,06 | | 0,15 |
| 12 | Sodium Chloride | | 0,01 | |
| 13 | Stearyl Alcohol | 4,00 | 3,80 | |
| 14 | Distearoylethyl Hydroxyethylmonium Methosulfate + Cetearyl Alcohol | 2,86 | | 2,86 |
| 15 | Oryzanol | 0,05 | 0,05 | 0,05 |
| 16 | PEG-90 M + Silica | 0,05 | 0,05 | |
| 17 | **Cyclomethicone** | **2,00** | | |
| 18 | Palmitamidopropyltrimonium Chloride + Propylene Glycol | 3,32 | 0,42 | 1,66 |
| 19 | **PPG-3 Benzyl Ether Myristate** | | **2,00** | **2,00** |
| 20 | Stearamidopropyl Dimethylamine | | 2,00 | |
| 21 | Parfum | 0,70 | 0,70 | 0,70 |
| **Table 1** | pH (90d) | 4,21 | 3,89 | 4,05 |
| | viscosity (90d) | 7400/4100 | 9100/6600 | 4900/3250 |

| **Tabelle 1** | | | | |
|---|---|---|---|---|
| **Handelsnamen der Rohstoffe** | | | | |
| 1 | Nipagin M, Clariant, 99.5% | | | |
| 2 | Nipasol M, Clariant, 99.5% | | | |
| 4 | Citric Acid Monohydrate Fine Granular 04 3277 6, DSM Nutritional Products Europe, 100% | | | |
| 5 | Nafol 1618-JA, Sasol , 100% | | | |
| 6 | Nacol 16-95, Sasol, 95% | | | |
| 7 | DC 200 Fluid 100cst, 100% | | | |
| 8 | 100366 Milchsäure 90, reinst DAB, Merck, 90% | | | |
| 9 | Uvasorb S5, 3V Sigma, 100% | | | |
| 13 | Lanette 18, Cognis, 100% | | | |
| 14 | Dehyquart F 75, Cognis, 69.935% | | | |
| 15 | Gamma-Oryzanol, Biesterfeld, 98% | | | |
| 16 | Polyox WSR-301 Leo NF Grade, Dow Chemical, 95% | | | |
| 17 | Dow Corning 245 Fluid, Dow Corning, 100% | | | |
| 18 | Varisoft PATC, Evonik Goldschmidt, 60% | | | |
| 19 | Crodamol STS, Croda, 99.95% | | | |
| 20 | Tego Amid S18, Evonik Goldschmidt, 100% | | | |

| In-Use-Test n=60 | | | |
|---|---|---|---|
| Test design: monadic | | | |
| Eigenschaft | 1 | 2 | 3 |
| angenehme Konsistenz | 5,2# | 5,3 | 5,4 |
| gute Kämmbarkeit nass | 5,5 | 5,7 | 5,6 |
| Fülle und Volumen | 4,0 | 4,3 | 4,5# |
| nicht fettiges Aussehen | 5,7 | 6,2 | 5,9 |
| beschwert Haar nicht | 5,6 | 5,7 | 5,8 |
| angenehmer Duft | 5,8 | 5,6 | 6,0 |
| Gesamturteil Qualität | 4,9 | 5,1 | 5,0 |
| besonders geeignet strap | 5,2 | 5,5 | 5,3 |

| **Tabelle 2** | | | |
|---|---|---|---|
| Signifikanz | # 20% | | |
| | * 10% | | |
| | ** 5% | | |

### Weitere Formel Beispiele:

**Tabelle 3**

| | **wt%** | **4** | **5** | **6** | **7** | **8** | **9** | **10** | **11** |
|---|---|---|---|---|---|---|---|---|---|
| 1 | Methylparaben | 0,25 | 0,25 | 0,25 | 0,25 | 0,25 | 0,25 | 0,25 | 0,25 |
| 2 | Propylparaben | 0,10 | 0,10 | 0,10 | 0,10 | 0,10 | 0,10 | 0,10 | 0,10 |
| 3 | Aqua | Ad. 100 | Ad. 100 | Ad.1 00 | Ad.1 00 | Ad.1 00 | Ad.1 00 | Ad.1 00 | Ad.1 00 |
| 4 | Citric Acid | | | | 0,01 | 0,01 | | 0,01 | |
| 5 | Cetearyl Alcohol | | | | 4,90 | 4,90 | 5,24 | 4,90 | |
| 6 | Cetyl Alcohol | 3,00 | 2,70 | 2,90 | | | | | 2,0 |
| 7 | Glycol Distearate | 1,00 | 1,00 | | | | | | |
| 8 | Lactic Acid | 0,78 | 0,78 | 0,78 | | | | | 0,70 |
| 9 | Bis-Diglyceryl Polyacyladipate-2 | | | | 2,00 | 2,00 | | 2,00 | |
| 10 | Glycerin | | | | 8,70 | 8,70 | 5,00 | 8,70 | |
| 11 | Sodium Hydroxide | | | | 0,03 | 0,03 | 0,15 | 0,03 | |
| 12 | Sodium Chloride | 0,01 | 0,01 | 0,01 | | | | | 0,01 |
| 13 | Hydroxypropyl Methylcellulose | | | | 0,25 | 0,25 | | 0,25 | |
| 14 | Stearyl Alcohol | 6,00 | 5,20 | 5,60 | | | | | 3,80 |
| 15 | Distearoylethyl Hydroxyethylmonium Methosulfate | | | | | | 2,86 | | |
| 16 | Cetrimonium Chloride | | | | 2,00 | 3,00 | | 2,00 | |
| 17 | Behentrimonium Chloride + Cetearyl Alcohol | | | | | | | 2,00 | |
| 18 | Oryzanol | 0,05 | 0,05 | 0,05 | 0,05 | 0,05 | 0,05 | 0,05 | 0,05 |
| 19 | Palmitamidopropyltrimonium Chloride + Propylene Glycol | | | | 2,00 | | 1,66 | | 0,5 |
| 20 | Coco Betaine | 0,85 | 0,85 | 0,85 | | | | | 0,85 |
| 21 | PPG-3 Benzyl Ether Myristate | 2,00 | 2,00 | 2,00 | 2,00 | 2,00 | 1,50 | 2,00 | 2,00 |
| 22 | Stearamidopropyl Dimethylamine | 2,20 | 2,20 | 2,20 | | | | | |
| 23 | Parfum | 0,70 | 0,70 | 0,70 | 0,50 | 0,50 | 0,70 | 0,50 | |

### Handelsnamen der Rohstoffe

- 1: Nipagin M, Clariant, 99.5%
- 2: Nipasol M, Clariant, 99.5%
- 5: Nafol 1618-JA, Sasol, 100%
- 6: Nacol 16-95, Sasol , 95%
- 7: Tegin G 1100, Evonik Goldschmidt, 90%
- 8: 100366 Milchsäure 90, reinst DAB, Merck, 90%
- 9: Softisan 649, Sasol, 100%
- 13: Methocel E4M Premium, Nordmann & Rassmann, 100%
- 14: Lanette 18, Cognis , 100%
- 15: Dehyquart F 75, Cognis, 69.935%
- 16: CTAC 6025 KC, KCI, 25%
- 17: Incroquat Behenyl TMC-25, Croda, 25%
- 18: Gamma -Oryzanol, Biesterfeld, 98%
- 19: Varisoft PATC, Evonik Goldschmidt, 60%
- 20: Dehyton AB 30, Cognis, 31%
- 21: Crodamol STS, Croda, 99.95%
- 22: Tego Amid S18, Evonik Goldschmidt, 100%

## Patentansprüche

1. Silikonfreie kosmetische Haarbehandlungszubereitung vorgesehen für das Ausspülen nach der Anwendung enthaltend PPG-3 Benzyl Ether Myristate **dadurch gekennzeichnet, dass** 0,0001 bis 1 Gew.-% amphotere Tenside enthalten sind.

2. Zubereitung nach Patentanspruch 1 **gekennzeichnet durch** einen Gehalt an PPG-3 Benzyl Ether Myristate von 0,5 bis 4 Gew.-%, bevorzugt 1 bis 3 Gew.-%.

3. Zubereitung nach einem der vorangehenden Patentansprüche **dadurch gekennzeichnet, dass** zusätzlich 0,1 bis 1 Gew.-% Oryzanol enthalten sind.

4. Zubereitung nach einem der vorangehenden Patentansprüche **dadurch gekennzeichnet, dass** die Zubereitung frei von Cellulosederivaten ist.

5. Zubereitung nach einem der vorangehenden Patentansprüche **dadurch gekennzeichnet, dass** die Zubereitung nicht waschaktiv ist.

6. Zubereitung nach einem der vorangehenden Patentansprüche **dadurch gekennzeichnet, dass** die Zubereitung als amphotere Tenside Betaine, ganz besonders bevorzugt Cocobetaine enthält.
